# EUROPEAN PATENT APPLICATION

(11) **EP 3 841 960 A1**
(43) Date of publication of application: **30.06.2021**
(21) Application number: 19219290.4
(22) Date of filing: 23.12.2019
(51) Int. Cl.: A61B 5/00

(54) **OPTIMIZING SLEEP ONSET BASED ON PERSONALIZED EXERCISE TIMING TO ADJUST THE CIRCADIAN RHYTHM**

(71) Applicant: Koninklijke Philips N.V., 5656 AG Eindhoven (NL)
(72) Inventor: VAN EE, Raymond, 5656 AE Eindhoven (NL); MENA BENITO, Estrella, 5656 AE Eindhoven (NL); LEUFKENS, Timmy Robertus Maria, 5656 AE Eindhoven (NL); WESTERINK, Joanne Henriëtte Desirée Monique, 5656 AE Eindhoven (NL); PFUNDTNER, Stefan, 5656 AE Eindhoven (NL)
(74) Representative: Philips Intellectual Property & Standards

(57) **Abstract**

The present invention relates to circadian rhythm management. In order to improve circadian rhythm management, an apparatus is provided that uses an alternative exercise-based concept and personalized approach for managing or controlling personal circadian rhythm and optimizing sleep onset.

## Description

### FIELD OF THE INVENTION

The present invention relates to circadian rhythm management. In particular, the present invention relates to a user device, a system, and a method for managing a circadian rhythm of a user.

### BACKGROUND OF THE INVENTION

The biological clock, stress and temperature influence sleep onset. It is well known that sunlight, and/or artificial light, is able to align the circadian output of a person with the solar circadian rhythm. Circadian pacing that is insufficiently synchronized with the environmental light-dark cycle (e.g., in jetlag or shift work) may dramatically influence sleep and sleep onset. However, sunlight and/or artificial light may not be always feasible for everyone for the reasons like bad weather, planned meetings or other inside activities, or early evening arrivals.

US 2019/0168018 A1 describes a method for managing circadian rhythm.

### SUMMARY OF THE INVENTION

There may be a need to improve circadian rhythm management.
The object of the present invention is solved by the subject-matter of the independent claims, wherein further embodiments are incorporated in the dependent claims. It should be noted that the following described aspects of the invention apply also for the user device, the system, and the method for managing a circadian rhythm of a user.

According to a first aspect of the present invention, there is provided a user device for managing a circadian rhythm of a user, comprising:
- an input unit,
- a processing unit, and
- an output unit.

The input unit is configured to receive data including information indicative of a circadian shift needed to the circadian rhythm of the user for at least one event. The processing unit is configured to determine whether the circadian shift exceeds a threshold, and to determine, upon a determination that the circadian shift exceeds the threshold, a target exercise timing for undertaking physical exercise based on the received data. The target exercise timing is a certain timing in a circadian phase at which undertaking physical exercise facilitates a shift in the circadian phase for accelerating the adaption of the circadian rhythm of the user to the needed circadian shift. The output unit is configured to output an exercise advice including the target exercise timing.

In other words, an alternative exercise-based concept is proposed for managing or controlling the personal circadian rhythm through determining adequate timing of physical exercise. Exercise at the proper time of the daily cycle can boost the amplitude of the rhythm of the suprachiasmatic nucleus (SCN) clock. People traveling through time-zones that experience jet-lag or people working out of sync with the light-dark cycle, e.g., shift workers, may be helped with a proper timing of exercise facilitating and accelerating the adaptation of the SCN to the desired time-zone, or light-dark cycle. In addition, when a person exercises at a certain time in the circadian phase, the rise in core temperature of the person due to exercise may facilitate a shift in circadian phase. If done at the correct timing, this shift may accelerate a person from reaching the correct, new circadian phase of the new location. The benefits of exercise and exercise-related signals in the brain can facilitate the synchronization of the internal circadian clock with the day-night cycle, thereby improving the circadian rhythm and sleep quality.

Various information may be used to indicate a circadian shift needed to the circadian rhythm of the user for at least one event. The at least one event may be a future event, e.g., a planned travel across time zones. For example, for time-zone travelers, e.g., from Amsterdam to New York, a change in time zone may indicate a circadian shift need to the circadian rhythm of the user for the time-zone travel. The at least one event may be a current event. For example, for people who have lost their circadian rhythm without time-zone travel, e.g. due to shift work or even circadian rhythm disorders, e.g., delayed sleep phase disorder, a desynchronization between the circadian rhythm of the user and the local light-dark cycle may be used to indicate that a circadian shift is needed for these people.

The user device may determine whether the circadian shift exceeds a threshold. For example, the user device may determine whether the change of time zone exceeds a threshold, e.g., three hours. For example, the user device may determine whether the desynchronization between the circadian rhythm of the user and the local light-dark cycle exceeds a threshold. For example, the core temperature of the user may be measured, e.g., by a temporary internal temperature sensor. If the lowest body temperature is between 3 and 5 am, no exercise advice will be provided. In other words, the circadian rhythm of the user is considered to align with the local light-dark cycle. If the lowest body temperature is out of this range, an exercise advice will be provided.

Upon a determination that the circadian shift exceeds the threshold, a target exercise timing for undertaking physical exercise is determined based on the received information. Exercise is a strong entrainment signal for mammalian circadian clock. Proper exercise can have a significant circadian phase-shifting effect, and affect the sleep and wake schedule in human. The correlation between the target exercise timing and the shift in the circadian phase may be derived from previously measured data, e.g., empirical findings of laboratory experiments. For example, a look up table may be used to map the information indicative of a circadian shift to a target exercise timing. For example, a simulation model may be used to map the information indicative of a circadian shift to a target exercise timing. The simulation model is parametrized based on exemplary pairs of information indicative of a circadian shift and the target exercise timing. For example, a machine learning model may be used to map the information indicative of a circadian shift to a target exercise timing. The machine learning model has been trained based on exemplary pairs of information indicative of a circadian shift and the target exercise timing.

The correlation may be affected by various factors. For example, a morning person and an evening person may have different target exercise timings. For example, people having different sleep patterns may have different target exercise timings. For example, people having different time schedules may have different target exercise timings. Therefore, it may be beneficial to include these factors for the determination of a proper exercise timing.

Furthermore, besides target exercise timing, the type of exercise (e.g., walking, jogging), the exercise duration (e.g., half-hour jogging), and exercise intensity (e.g., faster jogging speed) may have an effect on the shift in the circadian phase. Therefore, there is also a correlation between the needed circadian shift and the type of exercise, the exercise duration, and the exercise intensity. Therefore, the previously mentioned look-up table, simulation model, and/or the machine learning model may be used to map the needed circadian shift to further exercise information, such as the target type of exercise, the target exercise duration, and/or the target exercise intensity.

Finally, an exercise advice including the target exercise timing may be output, e.g. to a display such that the user can follow the exercise advice for undertaking physical exercise. Besides the target expertise timing, the exercise advice may also include at least one of a target type of exercise, a target exercise duration, and a target exercise intensity, in combination with the target exercise timing.

The user device may be any device capable of processing the information. In an example, the user device may be a mobile phone. In an example, the user device may be a smart watch. In an example, the user device may be a wearable computer eyeglass.

Accordingly, the user device may help people to re-establish their circadian rhythm without the use of light, including sunlight and artificial light. Instead, the personal circadian rhythm is managed or controlled through adequate timing of physical exercise, optionally in combination with adequate duration, intensity, and/or type of physical exercise, suggested by the provided user device.

According to an embodiment of the present invention, the processing unit is configured to determine a target exercise intensity, a target exercise duration, and/or a target type of exercise for undertaking physical exercise based on the received data to facilitate the shift in the circadian phase. The exercise advice further comprises the target exercise intensity, the target exercise duration, and/or the target type of exercise.

For example, the user device may provide a target intensity exercise based on the combination of the received information. In addition, the user device may utilize information about exercise-preference, namely the target type of exercise, to personalize the kind of physical sport or exercise. It may be also relevant to take into account whether a person prefers an entertaining team sport, e.g., soccer, or an individual concentration-demanding sport, e.g., weight lifting. This information may be used for personalized coaching.

According to an embodiment of the present invention, the information comprises a change in time-zone. The processing unit is further configured to determine the exercise advice as a function of the change in time zone.

Adjusting the exercise advice as a function of the change in time zone may be beneficial for time-zone travelers. The user device may determine how many hours the circadian rhythm needs to be shifted and in which direction. The user device may then calculate the optimal moments for exercise, such that the exercise will foster a quick re-establishment of the circadian rhythm and an alignment with the day-night cycle at the location of destination.

According to an embodiment of the present invention, the information comprises a local light-dark cycle and a currently measured circadian rhythm of the user. The processing unit is further configured to compare the currently measured circadian rhythm of the user and the local light-dark cycle to determine the circadian shift. The processing unit is further configured to determine the exercise advice as a function of the determined circadian shift for aligning the circadian rhythm of the user with the local light-dark cycle.

This may be beneficial for shift-workers and other persons with a disrupted circadian rhythm, e.g., a delayed sleep phase disorder, at a constant geographical location. The adjustment of the exercise advice may facilitate the synchronization of the internal circadian clock with the local day-night cycle, thereby improving the circadian rhythm and sleep quality.

According to an embodiment of the present invention, the processing unit is further configured to compare the currently measured circadian rhythm of the user and the local light-dark cycle to determine whether the user is a morning person or an evening person. The processing unit is further configured to adjust the exercise advice as a function of the determination.

Based on the result of the comparison between the internal diurnal rhythm and the world's day/night rhythm, the target exercising timing may be adapted. For example, this may be used before the time-zone trip, and upon arrival. The reason to already start before the trip lies in gradually modifying the circadian rhythm of the user.

According to an embodiment of the present invention, the data further comprises a sleep pattern of the user or an expected time of sleeping. The processing unit is configured to adjust the exercise advice as a function of the sleep pattern of the user or the expected time of sleeping.

Individual's sleep pattern or expected time of sleeping may also used to determine an optimal time for undertaking exercise.

According to an embodiment of the present invention, the information further comprises at least one real-time measured sleep parameter representative of a sleep quality. The processing unit is configured to adjust the exercise advice as a function of the at least one real-time measured sleep parameter.

Dynamical and automatic adaption of a target exercise may be done according to the individual outcome or results of initially proposed target exercise by means of real time measurements of the sleep quality, thus determining whether the personalized timing and intensity of exercise followed were successful. This information may be used to determine whether continued exercises are needed, and if so, at which times and intensities. It may also be used to ensure that the proposed exercises do not hamper sleep onset.

According to an embodiment of the present invention, the data further comprises at least one previously provided exercise advice and at least one sleep quality measured after undertaking physical exercise following the at least one previously provided exercise advice. The processing unit is configured to determine an effectiveness of the at least one previously provided exercise advice based on the at least one measured sleep quality. The processing unit is configured to adjust the exercise advice as a function of the effectiveness of the at least one previously provided exercise advice.

The sleep quality measurements may allow for adaptation of the exercise advice in future uses of the application, since the exercise advice given previously can be evaluated in terms of its effectiveness. Sleep quality may be important, since it is a night-time measure for how well the circadian rhythm has adapted, and as such may be an important indicator for how awake people feel during daytime. The sleep quality may be used as output measures in Artificial Intelligence (AI) techniques to calculate the optimal exercise moments and strengths based on the input and other measurement factors, such as solar cycle, circadian rhythm measurements, as usual, but in combination with the past effectiveness of the advice.

According to an embodiment of the present invention, the data further comprises a context of a patient including at least one of: a health history of the patient, a current activity of the patient, and a physical state of the patient. The processing unit is further configured to adjust the exercise advice as a function of the context of the patient.

According to an embodiment of the present invention, the data further comprises a time schedule of the user. The processing unit is further configured to adjust the exercise advice as a function of the time schedule of the user.

For example, the user device may take into account the agenda, timing of meals, etc, for determining the optimal moment to do exercise.

According to an embodiment of the present invention, the data further comprises demographics of the user. The processing unit is configured to adjust the exercise advice as a function of the demographics of the user.

Demographics is the collection and analysis of broad characteristics about groups of people and populations. The common variables that are gathered in demographic research may include age, sex, race, location, and level of education. Additional demographic factors may include gathering data on preferences, hobbies, lifestyle and more. For example, demography of sleep disturbances associated with circadian rhythm disorders in Japan may be different from that in the USA. For example, there are more evening persons in New York, whereas there are more morning persons in New Mexico. Hence, it may be beneficial to take demographic variables into consideration when mapping the needed circadian shift to the exercise advice.

According to an embodiment of the present invention, the processing unit is configured to apply a predictive data driven model to the received information indicative of a circadian shift needed to the circadian rhythm of the user for at least one event to estimate the exercise advice. The predictive data driven model is parametrized or trained according to a training dataset. The training dataset is based on sets of historical data comprising examples of the information and examples of the exercise advice.

According to an embodiment of the present invention, the user device further comprises a user interface configured to provide a warning signal to alert the user when the user need to start and finish exercising.

For example, the warning signal may be visual or auditory.

According to a second aspect of the present invention, there is provided a system for managing a circadian rhythm of a user, comprising:
- a sensor arrangement, and
- a user device according to the first aspect and any associated example.

The sensor arrangement comprises one or more sensors connectable to the user for providing sensor data to the user device.

In an example, the sensor arrangement may comprise one or more sensors worn by or connected to the user, such as a smart watch or a mobile phone.

In an example, the sensor arrangement may comprise a sensor for measuring the body temperature, e.g., by means of a temporary internal temperature sensor, ambient temperature, and/or local light-dark cycle.

In an example, the sensor arrangement may comprise a sensor to measure physiological values to determine the circadian rhythm.

In an example, the sensor arrangement may comprise a sensor to measure relevant sleep parameters, such as sleep onset latency and in general sleep quality.

According to a third aspect of the present invention, there is provided a method for managing a circadian rhythm of a user, comprising:
a) receiving, via an input channel, data comprising information indicative of a circadian shift needed to the circadian rhythm of the user for at least one event;
b) determining, via a processor, whether the circadian shift exceeds a threshold;
c) determining, via the processor, upon a determination that the circadian shift exceeds a threshold, a target exercise timing for undertaking physical exercise based on the received data, wherein the target exercise timing is a certain timing in a circadian phase at which undertaking physical exercise facilitates a shift in the circadian phase for accelerating the adaption of the circadian rhythm of the user to the needed circadian shift; and
d) outputting, via an output channel, an exercise advice including the target exercise timing.

According to an embodiment of the present invention, there is provided a computer program element controlling a user device as previously described which, if the computer program element is executed by a processing unit, is adapted to perform the method steps as previously described.

According to an embodiment of the present invention, there is provided a computer readable medium having stored computer element as previously described.

Advantageously, the benefits provided by any of the above aspects and examples equally apply to all of the other aspects and examples and vice versa.

As used herein, the expression "adjusting the exercise advice" may refer to calibrating the exercise advice. For example, the user device may map the information indicative of a needed circadian shift to a target exercise timing. Individual sleep patterns, individual time schedule, etc., may be added afterwards for adapting the target exercise timing to a personalized exercise timing. The expression "adjusting the exercise advice" may also refer to a process, which maps information indicative of a needed circadian shift and factors, such as individual sleep patterns, individual time schedule, etc., directly to a personalized exercise timing.

As used herein, the term "unit" may refer to, be part of, or include an Application Specific Integrated Circuit (ASIC), an electronic circuit, a processor (shared, dedicated, or group) and/or memory (shared, dedicated, or group) that execute one or more software or firmware programs, a combinational logical circuit, and/or other suitable components that provide the described functionality

These and other aspects of the present invention will become apparent from and be elucidated with reference to the embodiments described hereinafter.

### BRIEF DESCRIPTION OF THE DRAWINGS

These and other aspects of the invention will be apparent from and elucidated further with reference to the embodiments described by way of examples in the following description and with reference to the accompanying drawings, in which
Fig. 1 schematically shows a user device for managing a circadian rhythm of a user according to some embodiments of the present disclosure.
Fig. 2 schematically shows a system for managing a circadian rhythm of a user according to some embodiments of the present disclosure.
Fig. 3 shows a flow diagram of a method for managing a circadian rhythm of a user.

It should be noted that the figures are purely diagrammatic and not drawn to scale. In the figures, elements which correspond to elements already described may have the same reference numerals. Examples, embodiments or optional features, whether indicated as non-limiting or not, are not to be understood as limiting the invention as claimed.

### DETAILED DESCRIPTION OF EMBODIMENTS

Sleep onset is the transition from wakefulness into sleep. The internal mechanisms that regulate the almost ceaseless cycles of sleep and wakefulness make up a remarkable system. However, a variety of internal and external factors can dramatically influence the balance of this sleep-wake system. The biological clock, stress and temperature are factors that affect sleep onset.

Circadian (i.e. approximately daily or 24 hour) rhythms are regulated by the brain's biological clock, a small structure that is part of the hypothalamus which is called the suprachiasmatic nucleus (SCN). A vital task of the SCN is to pace physiological processes in a circadian rhythm that is synchronized with the environmental light-dark cycle. This allows for anticipation of time-of-day-dependent demand on resources involved in, for instance, sensory alertness and the immune system. Circadian pacing that is insufficiently synchronized with the environmental light-dark cycle (e.g., in jetlag or shift work) is known to disturb mood, well-being, and health.

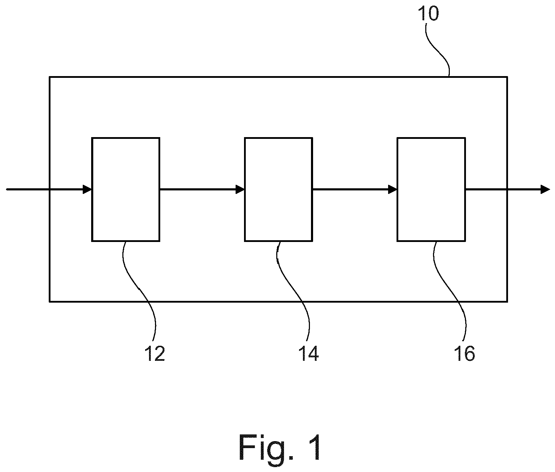

Fig. 1 schematically shows a user device 10 for managing a circadian rhythm of a user according to some embodiments of the present disclosure. The user device 10 may be a mobile device, which may include any type of wireless device, such as consumer electronics devices, smart phones, tablet personal computers, wearable computing devices, personal digital assistants (PDAs), laptop computers, and/or any other like physical computing device that is able to connect to a communications network.

The user device 10 comprises an input unit 12, a processing unit 14, and an output unit 16. The input unit 12 is configured to receive data including information indicative of a circadian shift needed to the circadian rhythm of the user for at least one event.

The processing unit 14 is configured to determine whether the circadian shift exceeds a threshold. The processing unit 14 is further configured to determine, upon a determination that the circadian shift exceeds the threshold, a target exercise timing for undertaking physical exercise based on the received information. The target exercise timing is a certain timing in a circadian phase at which undertaking physical exercise facilitates a shift in the circadian phase for accelerating the adaption of the circadian rhythm of the user to the needed circadian shift.

Optionally, the processing unit 14 may be configured to determine a target exercise intensity, a target exercise duration, and/or a target type of exercise for undertaking physical exercise based on the received information to facilitate the shift in the circadian phase. In other words, the correlation between the needed circadian shift and the target exercise intensity, the target exercise duration, and/or the target type of exercise for undertaking physical exercise may also be derived from previously measured data, e.g., empirical findings of laboratory experiments.

Optionally, the processing unit 14 may be configured to apply a predictive data driven model to the received information indicative of a circadian shift needed to the circadian rhythm of the user for at least one event to estimate the exercise advice. The predictive data driven model is parametrized or trained according to a training dataset. The training dataset is based on sets of historical data comprising examples of the information and examples of the exercise advice.

In an example, the predictive data driven model may include a simulation model to map the information indicative of a circadian shift to the exercise advice. The simulation model is parametrized by fitting the simulation model to exemplary pairs of information indicative of a circadian shift and the exercise advice.

In an example, the predictive data driven model may include a machine learning model. The machine learning model has been trained based on exemplary pairs of information indicative of a circadian shift and the exercise advice. As used herein, the term "machine learning" may refer to a statistical method that enables machines to "learn" tasks from data without explicitly programming. Machine learning techniques may comprise "traditional machine learning" - the workflow in which one manually selects features and then trains the model. Examples of traditional machine learning techniques may include decision trees, support vector machines, and ensemble methods. In some examples, the data driven model may comprise a data driven deep learning model. Deep learning is a subset of machine learning modeled loosely on the neural pathways of the human brain. Deep refers to the multiple layers between the input and output layers. In deep learning, the algorithm automatically learns what features are useful. Examples of deep learning techniques may include convolutional neural networks (CNNs), recurrent neural networks (such as long short-term memory, or LSTM), and deep Q networks. A general introduction into machine learning and corresponding software frameworks is described in "Machine Learning and Deep Learning frameworks and libraries for large-scale data mining: a survey"; Artificial Intelligence Review; Giang Nguyen et al., June 2019, Volume 52, Issue 1, pp 77-124.

The output unit 16 is configured to output an exercise advice including the target exercise timing. For example, the output unit 16 may output an exercise advice to a display, such as a display on a mobile phone or a display on a smart watch. For example, the output unit 16 may output the exercise advice to a calendar app of a mobile phone of the user. For example, the output unit 16 may output the exercise advice as a warning signal to alert the user when the user need to start and finish exercising.

Optionally, the exercise advice may further comprise the target exercise intensity, the target exercise duration, and/or the target type of exercise.

In an example, the information may comprise a change in time-zone. For time-zone travelers the information needed may comprise:
- Information about the current circadian rhythm of the user. This may be obtained from measurement of a core body temperature variation during the day, e.g. by means of a temporary internal temperature sensor. If can also be assumed that the current circadian rhythm is aligned with the geographical location of depart.
- Information about the new circadian rhythm needed, which is most likely determined by the geographical location of the destination.

The processing unit may then take these two inputs and determine how many hours the circadian rhythm needs to be shifted and in which direction. It may then calculate the optimal moments for exercise e.g., using a look-up table, a simulation model, or a machine learning model, such that the exercise will foster a quick re-establishment of the circadian rhythm and an alignment with the day-night cycle at the location of destination. While during the first day of time-zone travel, it might be reasonable to assume that the user's circadian rhythm coincides with that at the location of departure, this may hold less for later days of time-zone adjustment. Therefore, the core-body temperature measurements may be measured for this situation. In addition, information about time-zone travelers may used, based upon data from the itinerary. For example, it can be advised to do exercise in the evening upon arrival when going westwards (e.g. Amsterdam to New York), and every morning when going eastward (e.g. New York to Amsterdam). The benefit of this timing is that it also increases body temperature, which in turn, helps to delay sleep onset, and to help becoming awake.

In an example, the information may comprise a local light-dark cycle and a currently measured circadian rhythm of the user. The processing unit 14 may be configured to compare the currently measured circadian rhythm of the user, and the local light-dark cycle to determine the circadian shift. The processing unit 14 may be further configured to adjust the exercise advice as a function of the determined circadian shift for aligning the circadian rhythm of the user with the local light-dark cycle. In an example, a sensing system may be provided to measure physiological values, e.g., activity, respiration rate, pulse rate, and pulse rate variability, to determine the circadian rhythm. In an example, a sensing system may be provided for measuring the body temperature, e.g. by means of a temporary internal temperature sensor, to determine the circadian rhythm. The processing unit may then take these two inputs and determine how many hours the circadian rhythm needs to be shifted and in which direction. It may then calculate the optimal moments for exercise e.g., using a look-up table, a simulation model, or a machine learning model, such that the exercise will foster a quick re-establishment of the circadian rhythm and an alignment with the local day-night cycle. This example may be beneficial for shift-workers and other persons with a disrupted circadian rhythm at a constant geographical location.

In an example, the processing unit 14 may be further configured to compare the currently measured circadian rhythm of the user and the local light-dark cycle to determine whether the user is a morning person or an evening person. The processing unit 14 may be further configured to adjust the exercise advice as a function of the determination.. This example may be used before the time-zone trip, and upon arrival. The reason to already start before the trip lies in gradually modifying the circadian rhythm of the user. A morning person, e.g. who usually goes up at 5 am, will have more trouble going eastwards from Amsterdam to New York than an evening person, e.g. who usually goes up at 9 am. Accordingly, it may help to shift the circadian rhythm of a morning person to being a later awaking person.

In an example, the information further comprises a sleep pattern of the user or an expected time of sleeping. The processing unit 14 is configured to adjust the exercise advice as a function of the sleep pattern of the user or the expected time of sleeping. The sleep pattern is s a biological rhythm that guides the body as to when it should sleep and when it should wake. It is one of the body's circadian rhythms and typically follows a 24-hour cycle, controlling the body's schedule for sleeping and waking. Individual's sleep pattern or expected time of sleeping may be used to determine an optimal exercise advice. It may comprise the measure of circadian rhythm with a threshold value.

In an example, the data may further comprise at least one previously provided exercise advice and at least one sleep quality measured after undertaking physical exercise following the at least one previously provided exercise advice. The processing unit is configured to determine an effectiveness of the at least one previously provided exercise advice based on the at least one measured sleep quality. The processing unit is configured to adjust the exercise advice as a function of the effectiveness of the at least one previously provided exercise advice. A sensor arrangement with one or more sensors may be used to measure relevant sleep parameters, such as sleep onset latency and in general sleep quality, since one of the goals of the re-established circadian rhythm is optimal sleep. For example, both the sleep quality and the core-body temperature may be measured, which allow for adaptation of the exercise advice in future uses of the application, since the advice given previously can be evaluated in terms of its effectiveness. Sleep quality may be important, since it is a night-time measure for how well the circadian rhythm has adapted, while the core-body temperature is a proxy measure for how well the circadian rhythm has adapted, and as such is an important indicator for how awake people feel during day time. Both may be used as output measures in Artificial Intelligence (AI) techniques with a pre-trained machine learning model to calculate the optimal exercise moments and/or strengths on the basis of the input, which may also include other measurement factors, such as solar cycle, circadian rhythm measurements, as usual, in combination with the past effectiveness of the advice given.

In an example, the data may further comprise a context of a patient including at least one of: a health history of the patient, a current activity of the patient, and a physical state of the patient. The processing unit 14 may be further configured to adjust the exercise advice as a function of the context of the patient. In other words, the user device may take into account the context of the patient, such as health history or current activity or physical state of the person, as these parameters may affect the determination of e.g., the target exercise duration, the target exercise intensity, and/or the target exercise timing.

In an example, the data may further comprise a time schedule of the user, such as agenda, timing of meals, etc. The processing unit 14 is further configured to adjust the exercise advice as a function of the time schedule of the user. For example, the target exercise intensity may be dependent on the timing of meals. The exercise advice may provide a less intensive physical exercise for undertaking exercise right after a meal.

In an example, the information may further comprise demographics of the user. The processing unit 14 may be configured to adjust the exercise advice as a function of the demographics of the user.

In an example, the user device may further comprise a user interface configured to provide a warning signal to alert the user when the user need to start and finish exercising. An example of the user interface is a display. The warning signal could be visual or auditory.

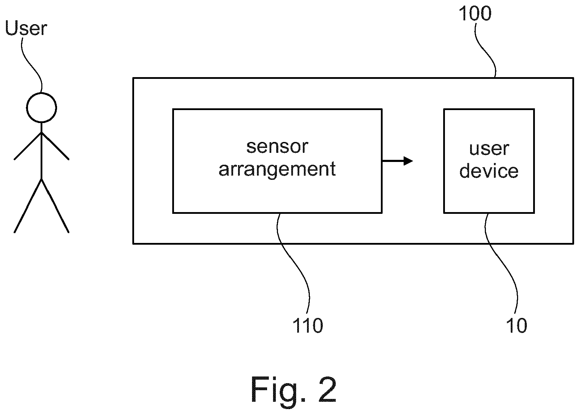

Fig. 2 schematically shows a system 100 for managing a circadian rhythm of a user according to some embodiments of the present disclosure. The system comprises a sensor arrangement 110 and a user device 10 as described above.

The sensor arrangement 110 comprises one or more sensors connectable to the user for providing sensor data to the user device 10. For example, the sensors may be integrated in a smart watch worn by the user.

In an example, the sensor arrangement 110 may comprise a sensor for measuring the body temperature, e.g. by means of a temporary internal temperature sensor. In an example, the sensor arrangement 110 may comprise a sensor for measuring an ambient temperature.

In an example, the sensor arrangement 110 may comprise a sensor for measuring a local light-dark cycle, e.g. by means of a photodetector.

In an example, the sensor arrangement 110 may comprise a sensor for measuring physiological values to determine the circadian rhythm. Examples of the physiological values include, but are not limited to, activity, respiration rate and RR variability, pulse rate and pulse rate variability.

In an example, the sensor arrangement 110 may comprise a sleep sensing system. This may be done by means of e.g. a wearable pulse oximeter for obtaining a photoplethysmogram (PPG) or a Smartsleep headband for obtaining the electroencephalography (EEG). These parameters may be important outcome measures, since one of the goals of the re-established circadian rhythm is optimal sleep.

The one or more sensors then output signal indicative of the sensed values or parameters to the user device as described above for managing a circadian rhythm of the user.

In an example, the system 100 may be built in a single device. For example, the sensor arrangement 110 may be integrated into the user device 10, such as a smart watch. In an example, the sensor arrangement 110 may be coupled to the user device 10 via a physical cable, e.g., USB cable, and/or wirelessly, e.g., via Bluetooth, for transmitting the sensor data to the user device 10.

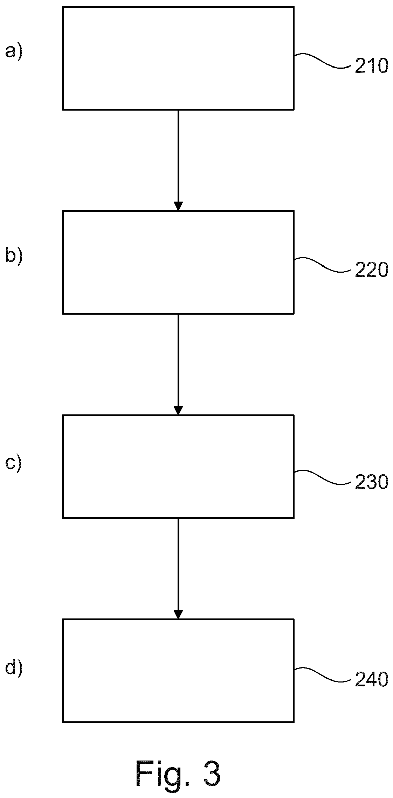

Fig. 3 shows a flow diagram of a method 200 for managing a circadian rhythm of a user. In step 210, i.e. step a) data comprising information indicative of a circadian shift needed to the circadian rhythm of the user for at least one event is received via an input channel.

In step 220, i.e. step b), it is determined by a processor whether the circadian shift exceeds a threshold.

In an example, step b) may further comprise determining whether a change of time zone exceeds a threshold. For example, the threshold may be one hour, two hours, or three hours, or any other value set by e.g., an operator at factory or by the user.

In an example, step b) may further comprise determining whether a desynchronization between the circadian rhythm of the user and the local light-dark cycle exceeds a threshold. For example, the threshold may be a time period between 3 and 5 am, or any other range set by e.g., an operator at factory or by the user.

In step 230, i.e. step c), it is further determined by the processor, upon a determination that the circadian shift exceeds a threshold, a target exercise timing for undertaking physical exercise based on the received data. The target exercise timing is a certain timing in a circadian phase at which undertaking physical exercise facilitates a shift in the circadian phase for accelerating the adaption of the circadian rhythm of the user to the needed circadian shift.

In an example, the information may further comprise a change in time-zone. step c) may further comprise determining the exercise advice as a function of the change in time zone.

In an example, the information may further comprise a local light-dark cycle and a currently measured circadian rhythm of the user. Step c) may further comprise comparing the currently measured circadian rhythm of the user and the local light-dark cycle to determine the circadian shift. Step c) may further comprise determining the exercise advice as a function of the determined circadian shift for aligning the circadian rhythm of the user with the local light-dark cycle.

In an example, step c) may further comprise comparing the currently measured circadian rhythm of the user and the local light-dark cycle to determine whether the user is a morning person or an evening person. Step c) may further comprise adjusting the exercise advice as a function of the determination.

In an example, the data further comprises a sleep pattern of the user or an expected time of sleeping. Step c) may further comprise adjusting the exercise advice as a function of the sleep pattern of the user or the expected time of sleeping.

In an example, the data further comprises at least one real-time measured sleep parameter representative of a sleep quality. Step c) may further comprise adjusting the exercise advice as a function of the at least one real-time measured sleep parameter.

In an example, the data may further comprise at least one previously provided exercise advice and at least one sleep quality measured after undertaking physical exercise following the at least one previously provided exercise advice. Step c) may further comprise determining an effectiveness of the at least one previously provided exercise advice based on the at least one measured sleep quality. Step c) may further comprise adjusting the exercise advice as a function of the effectiveness of the at least one previously provided exercise advice.

In an example, the data may further comprise a context of a patient including at least one of: a health history of the patient, a current activity of the patient, and a physical state of the patient. Step c) may further comprise adjusting the exercise advice as a function of the context of the patient.

In an example, the data may further comprise a time schedule of the user. Step c) may further comprise adjusting the exercise advice as a function of the time schedule of the user.

In an example, the data may further comprise demographics of the user. Step c) may further comprise adjusting the exercise advice as a function of the demographics of the user.

In an example, in step c), a predictive data driven model is applied to the received data comprising information indicative of a circadian shift needed to the circadian rhythm of the user for at least one event to estimate the exercise advice. The predictive data driven model is parametrized or trained according to a training dataset. The training dataset is based on sets of historical data comprising examples of the information and examples of the exercise advice.

In step 240, i.e. step d), an exercise advice including the target exercise timing is output.

In an example, step d) may further comprise determining a target exercise intensity, a target exercise duration, and/or a target type of exercise for undertaking physical exercise based on the received data to facilitate the shift in the circadian phase. The exercise advice further comprises the target exercise intensity, the target exercise duration, and/or the target type of exercise.

Optionally, the method may further comprise providing a warning signal to alert the user when the user need to start and finish exercising.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A user device (10) for managing a circadian rhythm of a user, comprising:
- an input unit (12);
- a processing unit (14); and
- an output unit (16);
wherein the input unit is configured to receive data including information indicative of a circadian shift needed to the circadian rhythm of the user for at least one event;
wherein the processing unit is configured to determine whether the circadian shift exceeds a threshold; and to determine, upon a determination that the circadian shift exceeds the threshold, a target exercise timing for undertaking physical exercise based on the received data, wherein the target exercise timing is a certain timing in a circadian phase at which undertaking physical exercise facilitates a shift in the circadian phase for accelerating the adaption of the circadian rhythm of the user to the needed circadian shift; and
wherein the output unit is configured to output an exercise advice including the target exercise timing.

2. User device according to claim 1,
wherein the processing unit is configured to determine a target exercise intensity, a target exercise duration, and/or a target type of exercise for undertaking physical exercise based on the received data to facilitate the shift in the circadian phase; and
wherein the exercise advice further comprises the target exercise intensity, the target exercise duration, and/or the target type of exercise.

3. User device according to claim 1 or 2,
wherein the information comprises a change in time-zone; and
wherein the processing unit is further configured to determine the exercise advice as a function of the change in time zone.

4. User device according to any one of the preceding claims,
wherein the information comprises a local light-dark cycle and a currently measured circadian rhythm of the user; and
wherein the processing unit is further configured to compare the currently measured circadian rhythm of the user and the local light-dark cycle to determine the circadian shift; and
wherein the processing unit is further configured to determining the exercise advice as a function of the determined circadian shift for aligning the circadian rhythm of the user with the local light-dark cycle.

5. User device according to claim 4,
wherein the processing unit is further configured to compare the currently measured circadian rhythm of the user and the local light-dark cycle to determine whether the user is a morning person or an evening person; and
wherein the processing unit is further configured to adjust the exercise advice as a function of the determination.

6. User device according to any one of the preceding claims,
wherein the data further comprises a sleep pattern of the user or an expected time of sleeping; and
wherein the processing unit is configured to adjust the exercise advice as a function of the sleep pattern of the user or the expected time of sleeping.

7. User device according to any one of the preceding claims,
wherein the data further comprises at least one real-time measured sleep parameter representative of a sleep quality; and
wherein the processing unit is configured to adjust the exercise advice as a function of the at least one real-time measured sleep parameter.

8. User device according to any one of the preceding claims,
wherein the data further comprises at least one previously provided exercise advice and at least one sleep quality measured after undertaking physical exercise following the at least one previously provided exercise advice;
wherein the processing unit is configured to determine an effectiveness of the at least one previously provided exercise advice based on the at least one measured sleep quality; and
wherein the processing unit is configured to adjust the exercise advice as a function of the effectiveness of the at least one previously provided exercise advice.

9. User device according to any one of the preceding claims,
wherein the data further comprises a context of a patient including at least one of:
- a health history of the patient;
- a current activity of the patient; and
- a physical state of the patient; and
wherein the processing unit is further configured to adjust the exercise advice as a function of the context of the patient.

10. User device according to any one of the preceding claims,
wherein the data further comprises a time schedule of the user; and
wherein the processing unit is further configured to adjust the exercise advice as a function of the time schedule of the user.

11. User device according to any one of the preceding claims,
wherein the data further comprises demographics of the user; and
wherein the processing unit is configured to adjust the exercise advice as a function of the demographics of the user.

12. User device according to any one of the preceding claims,
wherein the processing unit is configured to apply a predictive data driven model to the received information indicative of a circadian shift needed to the circadian rhythm of the user for at least one event to estimate the exercise advice, wherein the predictive data driven model is parametrized or trained according to a training dataset, wherein the training dataset is based on sets of historical data comprising examples of the information and examples of the exercise advice.

13. User device according to any one of the preceding claims, further comprising:
- a user interface configured to provide a warning signal to alert the user when the user need to start and finish exercising.

14. A system for managing a circadian rhythm of a user, comprising:
- a sensor arrangement; and
- a user device according to any one of the preceding claims;
wherein the sensor arrangement comprises one or more sensors connectable to the user for providing sensor data to the user device.

15. A method (200) for managing a circadian rhythm of a user, comprising:
a) receiving (210), via an input channel, data comprising information indicative of a circadian shift needed to the circadian rhythm of the user for at least one event;
b) determining (220), by a processor, whether the circadian shift exceeds a threshold;
c) determining (230), by the processor, upon a determination that the circadian shift exceeds a threshold, a target exercise timing for undertaking physical exercise based on the received information, wherein the target exercise timing is a certain timing in a circadian phase at which undertaking physical exercise facilitates a shift in the circadian phase for accelerating the adaption of the circadian rhythm of the user to the needed circadian shift; and
d) outputting (240), via an output channel, an exercise advice including the target exercise timing.
